# EUROPÄISCHE PATENTANMELDUNG

(11) **EP 2 789 600 A1**
(43) Veröffentlichungstag der Anmeldung: **15.10.2014**
(21) Anmeldenummer: 13163052.7
(22) Anmeldetag: 10.04.2013
(51) Int. Cl.: C07C 51/083, C07C 51/373

(54) **Verfahren zur Herstellung von ortho-acylierten aromatischen oder heteroaromatischen Carbonsäuren**

(71) Anmelder: Saltigo GmbH, 51369 Leverkusen (DE)
(72) Erfinder: Alain, Cotté, 51377 Leverkusen (DE); Lukas, Goossen, 67663 Kaiserslautern (DE); Patrizia, Mamone, 67269 Grünstadt (DE); Grégory, Damoun, 28210 Villemeux sur Eure (FR)
(74) Vertreter: Pettrich, Klaus-Günter

(57) **Zusammenfassung**

Beschrieben wird ein Verfahren zur Herstellung ortho-acylierter aromatischer oder heteroaromatischer Carbonsäuren, bei dem man eine aromatische oder heteroaromatische Carbonsäure oder ihr Anhydrid, die jeweils über eine CH-Gruppe in ortho-Position zur Carboxylatfunktion verfügt, in Gegenwart eines Rhodiumkatalysators und einen basischen Additivs mit einem Acylierungsreagenz umsetzt.

## Beschreibung

Die Erfindung betrifft ein Verfahren zur Herstellung einer ortho-acylierten aromatischen oder heteroaromatischen Carbonsäure, bei dem eine aromatische oder heteroaromatische Carbonsäure mit einem Acylierungsreagenz in Gegenwart einen Rhodiumkatalysators in der ortho-Stellung zur Carboxylgruppe acyliert wird.

Ortho-acylierte aromatische Carbonsäuren sind wichtige Grundkörper in der organischen Synthese. Die o-Acylbenzoesäure Balanol ist beispielsweise ein wirksamer Proteinkinase1 C Inhibitor (siehe z.B. A. K. Srivastava, G. Panda, Chem. Eur. J. 2008, 14, 4675-4688.), ähnliche Verbindungen sind Schlüsselintermediate bei der Synthese von 2-[2-(Imidazolyl)alkyl]-1(2H)-phthalazinonen mit Antiasthma-Aktivität (siehe z.B. M. Yamaguchi, K. Kamei, T. Koga, M. Akima, T. Kuroki, N. Ohi, J. Med. Chem. 1993, 36, 4052-4060.) oder von anxiolytischen Isoindolinonderivaten (siehe z. B. S. M. Allin, C. J. Northfield, M. I. Page, A. M. Z. Slawin, J. Chem. Soc., Perkin Trans. 1 2000, 1715-1721.).

Traditionelle Zugänge zu diesem Verbindungsklassen wie beispielsweise die Ringöffnung von Phthalsäureanhydriden sind entweder unselektiv (Friedel Crafts Reaktionen (siehe hierzu beispielsweise H. Yu, Y. Xiao, H. Guo, Org. Lett. 2012, 14, 2014-2017.)) oder erfordern empfindliche und teure organometallische Reagenzien (siehe hierzu beispielsweise W. E. Parham, C. K. Bradsher, K. J. Edgar, J. Org. Chem. 1981, 46, 1057-1061).

Die Friedel-Crafts-Acylierung von aromatischen Carbonsäuren oder ihrer Derivate ist nur unter harschen Bedingungen möglich, wobei die Acylgruppe selektiv in meta-Position zu Carboxylatgruppe eingeführt wird.

Es bestand daher Bedarf an einem katalytischen Verfahren, mit dem Acylgruppen selektiv in die ortho-Position aromatischer Carbonsäuren eingeführt werden können.

Es sind bisher jedoch keine Katalysatoren bekannt, die die Acylierung aromatischer Carbonsäuren mit gängigen Acylierungsreagenzien, wie z.B. Acylhalogeniden oder Carbonsäureanhydriden, selektiv in die ortho-Position der Carboxylatgruppe dirigieren.

Es ist lediglich bekannt, dass aromatische Moleküle mit stark koordinierenden Gruppen in Gegenwart bestimmter Übergangsmetallkatalysatoren selektiv in ortho-Positionen acyliert werden. Dabei werden überwiegend Aldehyde als Acylierungsreagenzien eingesetzt (siehe hierzu beispielsweise X. Jia, S. Zhang, W. Wang, F. Luo, J. Cheng, Org. Lett. 2009, 11, 3120-3123). Die begrenzte Verfügbarkeit und die Oxidationsempfindlichkeit dieser Verbindungsklasse limitiert jedoch den präparativen Nutzen dieser Methoden.

Auch Alpha-Oxocarbonsäuren wurden bereits als Acylierungsreagenzien verwendet (siehe hierzu beispielsweise M. Li, H. Ge, Org. Lett. 2010, 12, 3464-3467). Diese Verbindungen sind jedoch nur schlecht verfügbar und die Acylierungen erfordern den Zusatz stöchiometrischen Mengen eines Oxidationsmittels.

Aromatische Verbindungen mit stark koordinierenden Pyridingruppen können auch durch Säurechloride (T. Kochi, A. Tazawa, K. Honda, F. Kakiuchi, Chem. Lett. 2011, 40, 1018-1020.) oder Säureanhydride (J. Lu, H. Zhang, X. Chen, H. Liu, Y. Jiang, H. Fu, Adv. Synth. Catal. 2013, 355, 529-536.) in ortho-Position acyliert werden, wobei Ruthenium-oder Palladiumkatalysatoren eingesetzt werden. Im Vergleich zu Pyridingruppen sind Carboxylatgruppen weitaus weniger stark koordinierend (siehe z.B. K. M. Engle, T.-S. Mei, M. Wasa, J.-Q. Yu, Acc. Chem. Res. 2012, 45, 788-802.). So gelang es bisher nicht, ähnliche Strategien auch für ortho-Acylierungen aromatischer Carbonsäuren nutzbar zu machen.

Der vorliegenden Erfindung lag daher die Aufgabe zu Grunde, ein Verfahren bereitzustellen, das die Herstellung ortho-acylierter aromatischer oder heteroaromatischer Carbonsäuren aus einfach verfügbaren aromatischen bzw. heteroaromatischen Carbonsäuren und gut verfügbaren Acylierungsreagenzien unter redoxneutralen Bedingungen in guten Ausbeuten erlaubt.

Es wurde jetzt ein Verfahren unter Einsatz eines Rhodiumkatalysators gefunden, das diese Aufgabe löst.

Die Erfindung betrifft daher ein Verfahren zur Herstellung einer ortho-acylierten aromatischen oder heteroaromatischen Carbonsäure der Formel (3), 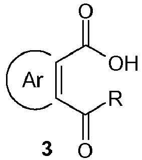 in der Ar für ein aromatisches oder heteroaromatisches Ringsystem steht,
durch Umsetzung einer Verbindung der Formel (1) 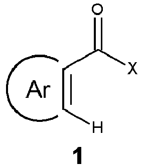 in der X für eine Hydroxylgruppe oder den Rest 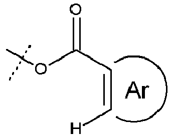 steht und Ar die für Formel (3) angegebene Bedeutung hat,
a) mit einem Acylierungsreagenz der Formel (2) 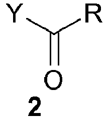 in der R für einen C₁-C₈- Alkylrest und
   Y für ein Chlor-, Brom-, lod- oder Fluoratom oder einen über ein Sauerstoffatom gebundenen Carboxylrest R¹COO, bei dem R¹ unabhängig von R die gleiche Bedeutung wie R besitzt, steht (Verfahrensvariante a))
   oder
b) mit einer weiteren Verbindung der Formel (I) in Form ihres Anhydrids (Verfahrensvariante b))
   in Gegenwart eines Rhodiumkatalysators und eines basischen Additivs.

Beim erfindungsgemäßen Verfahren zur Herstellung ortho-acylierter aromatischer oder heteroaromatischer Carbonsäuren, wird also eine aromatische oder heteroaromatische Carbonsäure oder ihr Anhydrid, die über eine C-H Gruppe in ortho-Position zur Carboxylatgruppe verfügt (Verbindung der Formel (1)) in Gegenwart eines Rhodiumkatalysators und eines basischen Additivs mit einem Acylierungsreagenz (Verbindung der Formel (2)) gemäß der allgemeinen Reaktionsgleichung (Schema 1) umgesetzt. (Variante a)). Das Acylierungsreagenz (2) kann wahlweise auch ein vor (Variante b)) oder während (Variante c)) der Reaktion generiertes Anhydrid der aromatischen bzw. heteroaromatischen Carbonsäure (1) sein. Dabei wird die aromatische bzw. heteroaromatische Carbonsäure in ortho-Position acyliert, und es bildet sich ein Gleichgewichtsgemisch von gemischten Anhydriden, substituierten Phthaliden und Carbonsäuren. Nach Hydrolyse dieses Gemisches wird eine ortho-acylierte aromatische bzw. heteroaromatische Carbonsäure erhalten.

Die Entdeckung, dass Rhodiumkatalysatoren eine einzigartige Aktivität für diese Umsetzung zeigen, war insofern überraschend, als die weitläufig verwandte ortho-Acylierung donorsubstituierter Arene mit Carbonsäurederivaten durch Palladium- und Rutheniumkatalysatoren, nicht aber durch Rhodiumkatalysatoren vermittelt wird.

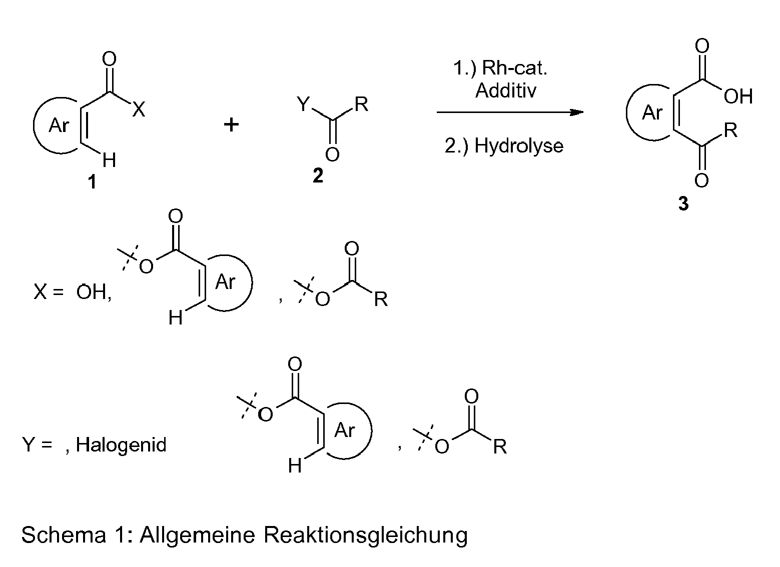

Da Carbonsäuren mit Acylierungsreagenzien (2) unweigerlich unter Bildung eines Gemisches von symmetrischen und unsymmetrischen Anhydriden reagieren, ergeben sich eine Reihe von mögliche Reaktionsvarianten dieser allgemeinen Reaktionsgleichung, von denen drei im Folgenden näher ausgeführt werden.
1) Variante a):Eine aromatische bzw. heteroaromatische Carbonsäure bzw. das Anyhdrid einer solchen Verbindung (Verbindung der Formel (1)) wird mit einem Acylierungsreagenz (Verbindung der Formel (2)), vorzugsweise ein aliphatisches Carbonsäureanhydrid oder -chlorid, umgesetzt. Dabei bildet sich zunächst ein Gemisch symmetrischer und unsymmetrischer Anhydride, das in Gegenwart des Katalysators zu einer aromatischen Carbonsäure mit einem ortho-Acylsubstituenten umgesetzt wird. 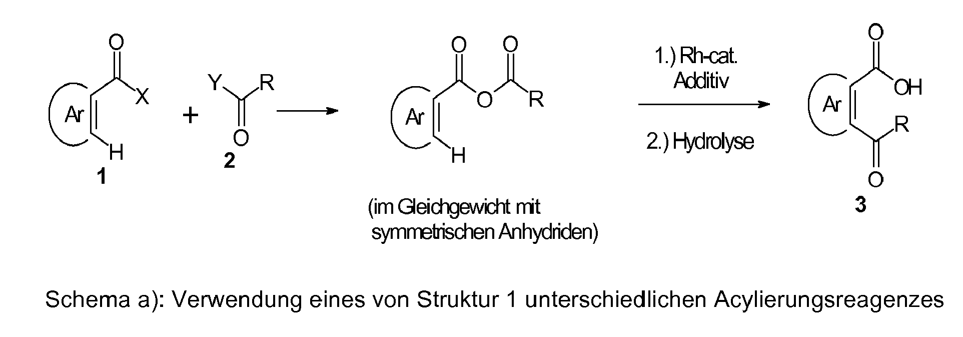 Bei dieser Verfahrensvariante beeinflusst der Substituent R am Acylierungsreagenz die Selektivät der Produktbildung. Wenn primäre oder sekundäre Alkylcarbonsäureanhydride oder -halogenide verwendet werden, wird selektiv ein Acylrest mit dem Substituenten R eingefügt. Die Bildung von ortho-acyliertem Produkt durch Acylierung der aromatischen Carbonsäure mit ihrem Anhydrid unterbleibt weitgehend. Werden dagegen tertiäre Alkylcarbonsäureanhydride oder -halogenide eingesetzt, so wird - wie in Reaktionsvariante c) beschrieben - ausschließlich ortho-acyliertes Produkt erhalten.
2) Variante b): Das Anhydrid einer aromatischen bzw. heteroaromatischen Carbonsäure wird gleichzeitig als Substrat (1) und als Acylierungsreagenz (2) eingesetzt, so dass als Produkt eine ortho-aroylierte aromatische bzw. heteroaromatische Carbonsäure (3a) entsteht. Diese Reaktionsvariante ist in Schema b) illustriert. 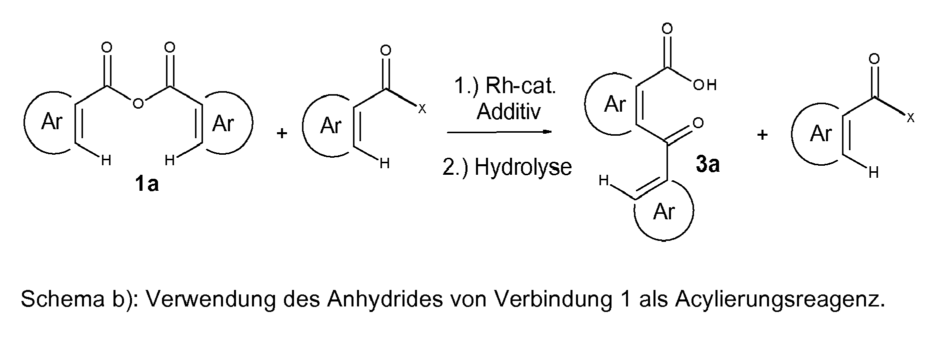
3) Variante c): Eine aromatische bzw. heteroaromatische Carbonsäure wird *in situ* durch ein Dehydratisierungsreagenz in ihr Anhydrid überführt, das nun gleichzeitig als Substrat (1) und als Acylierungsreagenz (2) dient, so dass als Produkt eine ortho-aroylierte aromatische bzw. heteroaromatische Carbonsäure entsteht. Diese Reaktionsvariante ist in Schema c) illustriert. Als Dehydratisierungsreagenz hat sich Pivalinsäureanhydrid besonders bewährt.

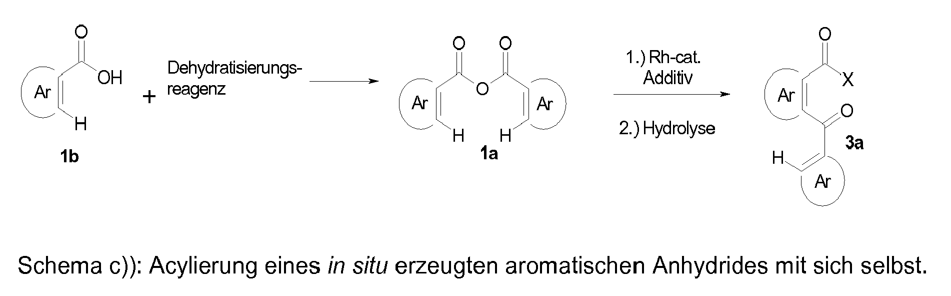

Eine bevorzugte Verfahrensvariante ist daher ein Verfahren, bei dem das Anhydrid der aromatischen oder heteroaromatischen Carbonsäure der Formel (1) *in situ* durch Zugabe eines Dehydratisierungsreagenz erzeugt wird (Verfahrensvariante c)).

Verfahrensvariante b) und c) sind dabei keine intramolekularen Umlagerungen sondern Rhodium-katalysierte intermolekulare Acylierungsreaktionen.

Verbindungen der Formel (1) sind also aromatische bzw. heteroaromatische Carbonsäuren oder deren Anhydride. Als aromatische Carbonsäuren können z.B. aromatische ein oder mehrkernige C₆-C₁₄-Carbonsäuren, z.B. Benzoesäure, 2-Tolylsäure, 3-Tolylsäure" 4-Tolysäure, Naphtylsäure, eingesetzt werden. Als heteroaromatische Carbonsäure können z.B. solche eingesetzt werden, die aus einem oder zwei oder drei anellierten 5- oder 6-gliedrigen aromatischen Ringen aufgebaut sind, worin in wenigstens einem Ring 1, 2, 3 oder 4 der Ringkohlenstoffatome durch ein Heteroatom, vorzugsweise ausgewählt aus den Elementen Sauerstoff, Stickstoff und Schwefel, ersetzt sind. Beispiele für heteroaromatische Carbonsäuren sind z.B. solche, die einen heteroaromatischen Kern aus der Gruppe Pyridyl, Chinolinyl, Acridinyl, Pyridazinyl, Pyrimidinyl, Pyrazinyl, Triazinyl, Tetrazinyl, Pyrrolyl, Pyrazolyl, Isoxazolyl, Imidazolyl, Oxazolyl, Thiazolyl, Thiophenyl, Furanyl enthalten.

Neben C₁-C₈-Alkylgruppen, z.B. 2-Tolylsäure können die aromatischen oder heteroaromatischen Carbonsäuren auch mit C₂-C₂₀-Vinyl-, C₆-C₂₀-Aryl-, oder C₃-C₂₀oder C₁-C₂₀-Alkoxy-Gruppen, z.B. Methoxy, am aromatischen bzw. heteroaromatischen Kern substituiert sein. Auch mit Hydroxy, Acyl, Amido, Amino, Cyano, Nitro, Fluor, Chlor, Brom, oder Iod substituierte Aromaten bzw. Heteroaromaten sind möglich. Weiterhin können 1,2,3 oder auch 4 Wasserstoffatome am Kern der aromatischen oder heteroaromatischen Carbonsäuren durch Halogenalkylgruppen substituiert sein. Beispiele für Halogenalkylgruppen sind Fluormethyl, Chlormethyl, Difluormethyl, Trifluormethyl, Trichlormethyl, 1-Fluorethyl, 2-Fluorethyl, 1,1-Difluorethyl, 2,2-Difluorethyl, 1,2-Difluorethyl, 2,2,2-Trifluorethyl, 1,1,2,2-Tetrafluorethyl, Pentafluorethyl.

Im erfindungsgemäßen Verfahren werden als Acylierungsreagenz Verbindungen der allgemeinen Formel (2) eingesetzt, 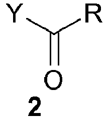 wobei R für einen C₁-C₈- Alkylrest steht und Y für ein Chlor-, Brom-, lod- oder Fluoratom oder einen über ein Sauerstoffatom gebundenen Carboxylrest R¹COO, bei dem R¹ unabhängig von R die gleiche Bedeutung wie R besitzt, steht. Steht Y für einen R¹COO-Rest, so ist das Acylierungsreagenz ein Carbonsäureanhydrid, das, wenn R gleich R¹ ist, ein symmetrisches Carbonsäureanhydrid darstellt, und, wenn R ungleich R¹ ist, ein unsymmetrisches Carbonsäureanhydrid darstellt.

Die Bezeichnung C₁-C₈-Alkyl soll dabei für einen geradkettigen oder bei mindestens drei Kohlenstoffatomen auch cyclischen oder verzweigten Alkylrest stehen.

C₁-C₈-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl , n-Octyl und Cyclohexyl.

Dabei kann der C₁-C₈-Alkylrest unsubstituiert sein oder einen oder mehrere, z.B. 1, 2 oder 3, Substituenten habeen, z.B., eine Hydroxy-, Mercapto-, Halogen-, Nitro-, Alkyl-Halogenalkyl-Alkoxy-, Halogenalkoxy-, Alkylthio-, Halogenalkylthio-, Alkylcarbonyl-, Alkylcarbonyloxy-, Alkylcarbonylthio-, Halogenalkylcarbonyl-, Alkoxycarbonyl- oder Cycloalkylgruppe.

Bevorzugt werden als Acylierungsreagenz symmetrische Carbonsäureanhydride der allgemeinen Formel (2) wie z.B. Essigsäureanhydrid oder Propionsäureanhydrid, eingesetzt,

Üblicherweise steht R in Formel (2) für einen C₁-C₈- Alkylrest. R kann aber auch für einen organischen Rest aus der Reihe A Alkenyl, , Heterocycloalkyl, Aryl, Hetaryl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl I stehen, der wahlweise unsubstituiert ist oder einen oder mehrere, z.B. 1, 2 oder 3, Reste aufweisen kann, die unter Hydroxy, Mercapto, Halogen, Nitro, Nitroso, Formyl, Alkyl, Halogenalkyl, Alkoxy, Halogenalkoxy, Alkylthio, Halogenalkylthio, Alkylcarbonyl, Alkylcarbonyloxy, Alkylcarbonylthio, Halogenalkylcarbonyl, Alkoxycarbonyl und Cycloalkyl ausgewählt sind.

Das Acylierungsreagenz wird bevorzugt im Überschuss eingesetzt, besonders bevorzugt in einer Menge von 2-5 Äquivalenten bezogen auf die aromatische bzw. heteroaromatische Carbonsäure.

Als Acylierungsreagenz können gemäß der Verfahrensvariante b) auch die aromatischen bzw. heteroaromatischen Carbonsäuresubstrate (1) selbst eingesetzt werden. Dazu werden sie direkt in Form ihrer Anhydride eingesetzt (Verfahrensvariante b)), oder gemäß Verfahrensvariante c) durch Zusatz gängiger Dehydratisierungsreagenzien *in situ* in Anhydride überführt. Beispiele für Dehydratisierungsreagenzien, die bekanntermaßen Carbonsäuren in Anhydride überführen, sind Sulfonsäurechloride, Phosphorpentoxid, Dialkylcarbodiimide, Trifluoressigsäureanhydrid, Dialkylcarbonate und Carbonsäureanhydride.

Bevorzugt werden als Dehydratisierungsreagenzien sterisch anspruchsvolle Carbonsäureanhydride eingesetzt, besonders bevorzugt wird Pivalinsäureanhydrid verwendet.

Als Katalysator werden gängige Rhodiumverbindungen eingesetzt. Wahlweise werden Rhodium(III) Verbindungen vorzugsweise aus der Gruppe Rhodiumchlorid, Rhodiumbromid, Rhodiumphosphat, Rhodiumoxid, Rhodiumnitrat, Rhodiumacetylacetonat, Rhodiumsulfat, Pentamethylcyclopentadienylrhodiumchlorid-Dimer,

Rhodium(II) Verbindungen vorzugsweise aus der Gruppe Rhodiumacetat, Rhodiumbenzoat Rhodiumpivalat und Rhodiumtrifluoracetat,
oder Rhodium(I) Verbindungen aus der Gruppe 1,5-Cyclooctadien-rhodium(I)chlorid Dimer, Bicyclo[2.2.1]hepta-2,5-dien-rhodium(I)chlorid Dimer, 2,5-Norbornadien-rhodium(I)chlorid Dimer, Di-µ-chlorobis[(1,2,5,6-η)-1,5-hexadien]dirhodium, Chlorobis(cycloocten)rhodium(I) Dimer, Chlorobis(ethylen)rhodium(I) Dimer, Acetylacetonatobis-(ethylen)rhodium(I), (Acetylacetonato)(bicyclo[2.2.1]hepta-2,5-dien)rhodium(I), (Acetylacetonato)(norbornadien)-rhodium(I), (Acetylacetonato)(1,5-cyclooctadien)rhodium(I), Bis(1,5-cyclooctadien)rhodium(I)-tetrafluoroborat, Bis(norbornadien)rhodium(I)tetrafluoroborat Bis(1,5-cyclooctadien)-rhodium(I)tetrakis[bis(3,5-trifluoromethyl)phenyl]borat, Bis-(acetonitril)(1,5-cyclooctadien)-rhodium(I)tetrafluoroborat, (1,5- Cyclooctadien)bis(triphenylphosphin)rhodium(I)hexafluoro-phosphat, Bis(1,5-cyclooctadien)rhodium(I)hexafluoroantimonat, [(Bisacetonitril)(norbornadien)]-rhodium(I)hexafluoroantimonat, Bis(norbornadien)rhodium(I)-trifluoromethansulfonat, Bis(1,5-cyclooctadien)rhodium(I)trifluoromethanesulfonat, Bis[(2,3,5,6-n)-bicyclo[2.2.1]hepta-2,5-dien]-rhodium-1,1,1-trifluoromethansulfonat, Hydroxy(cyclooctadien)rhodium(I) Dimer, Methoxy(cyclooctadien)rhodium(I) Dimer, (1,5-Cyclooctadien)(8-chinolinolato)rhodium(I), TMEDA(1,5-cyclooctadien)rhodium(I), Di-carbonyl(pentamethylcyclopentadienyl)rhodium(I), (Acetylacetonato)dicarbonylrhodium(I), Di-p-chloro-tetracarbonyldirhodium(I), Carbonyltris-(triphenylphosphin)rhodium(I)hydrid, Hydridotetrakis(triphenylphosphin)rhodium(I), Tris(triphenylphosphin)rhodium(I)chlorid, Bis(triphenylphosphin)rhodium(I)carbonylchlorid, Nitrosyltris(triphenylphosphin)rhodium(I), Hydroxy[-(S)-BINAP]-rhodium(I) Dimer eingesetzt.

Bevorzugt werden Rhodium(I)komplexe mit Olefinliganden, insbesondere aus der Gruppe

1,5-Cyclooctadien-rhodium(I)chlorid Dimer, Bicyclo[2.2.1]hepta-2,5-dien-rhodium(I)chlorid Dimer, 2,5-Norbornadien-rhodium(I)chlorid Dimer, Di-µ-chlorobis[(1,2,5,6-η)-1,5-hexadien]dirhodium, Chlorobis(cycloocten)rhodium(I) Dimer, Chlorobis(ethylen)rhodium(I) Dimer, Acetylacetonatobis(ethylen)rhodium(I), (Acetylacetonato)(bicyclo[2.2.1]hepta-2,5-dien)rhodium(I), (Acetylacetonato)(norbornadien)-rhodium(I), (Acetylacetonato)(1,5-cyclooctadien)rhodium(I), Bis(1,5-cyclooctadien)rhodium(I)-tetrafluoroborat, Bis(norbornadien)rhodium(I)tetrafluoroborat Bis(1,5-cyclooctadien)-rhodium(I)tetrakis[bis(3,5-trifluoromethyl)phenyl]borat, Bis(acetonitril)(1,5-cyclooctadien)-rhodium(I)tetrafluoroborat, Bis(1,5-cyclooctadien)rhodium(I)hexafluoroantimonat, [(Bisacetonitril)(norbornadien)]rhodium(I)-hexafluoroantimonat, Bis(norbornadien)rhodium(I)trifluoromethansulfonat, Bis(1,5-cyclooctadien)rhodium(I)trifluoromethansulfonat, Bis[(2,3,5,6-n)-bicyclo[2.2.1]hepta-2,5-dien]-rhodium-1,1,1-trifluoromethansulfonat, Hydroxy(cyclooctadien)rhodium(I) Dimer, Methoxy(cyclooctadien)rhodium(I) Dimer, (1,5-Cyclooctadien)(8-chinolinolato)rhodium(I), TMEDA(1,5-cyclooctadien)rhodium(I) eingesetzt.

Besonders bevorzugt werden Rhodium(I)komplexe mit Olefinliganden aus der Gruppe 1,5-Cyclooctadien-rhodium(I)chlorid Dimer, Di-µ-chlorobis[(1,2,5,6-η)-1,5-hexadien]dirhodium, Chlorobis(cycloocten)rhodium(I) Dimer und Chlorobis(ethylen)rhodium(I) Dimer verwendet.

Beim erfindungsgemäßen Verfahren beträgt die Katalysatormenge 0,001 mol% bis 20 mol% bezogen auf die aromatische bzw, heteroaromatische Carbonsäure. Vorzugsweise beträgt die Katalysatormenge 0,01 mol% bis 3 mol%

Als basische Additive werden tertiäre Amine wie z.B. Trethylamin oder anorganische Basen eingesetzt. Bevorzugt werden anorganische Salze mit Gegenionen wie z.B. Fluorid, Carbonat, Bicarbonat, Phosphat, Hydrogenphosphat, Dihydrogenphosphat, Pivalat, Acetat verwendet.

Besonders bevorzugt werden Metalfluoride, Phosphate und Carbonate eingesetzt, ganz besonders bevorzugt aus der Gruppe Kaliumfluorid, Cäsiumfluorid, Lithiumcarbonat, Natriumcarbonat, Kaliumcarbonat, Cäsiumcarbonat, Kaliumhydrogencarbonat, Natriumphosphat, Kaliumphosphat, Cäsiumphosphat, Kaliumhydrogenphosphat.

Beim erfindungsgemäßen Verfahren wird das Additiv in einer Menge von 10 mol% bis 500 mol% bezogen auf die aromatische Carbonsäure eingesetzt. Vorzugsweise wird es in einer Menge von 20 mol% bis 200 mol% eingesetzt.

Das erfindungsgemäße Verfahren wird bei Temperaturen von 20 °C bis 200 °C, vorzugsweise bei 80 °C bis 180 °C und besonders bevorzugt bei 120 °C bis 155 °C durchgeführt.

Das erfindungsgemäße Verfahren kann in Gegenwart eines Lösungsmittels oder in Substanz, d.h. ohne Lösungsmittel durchgeführt werden. Beispielsweise können als Lösungsmittel das Acylierungsreagenz selbst oder , Hexan, Heptan, Octan, Cyclohexan, Benzol, Toluol, Xylole, Ethylbenzol, Mesitylen, Dioxan, Tetrahydrofuran, Diethylether, Dibutylether, Methyl-*t*-butylether, Diisopropylether, Diphenylether, Diethylenglycoldimethylether, Methanol, Ethanol, Propanol, Isopropanol, Methylacetat, Ethylacetat, t-Butylacetat, Cyclohexanon, Dimethylformamid, Diethylformamid, N-Methylpyrrolidon, Dimethylacetamid, Dimethylsulfoxid, Sulfolan, Acetonitril, Propylencarbonat, Propionitril, oder chlorierte Kohlenwasserstoffe eingesetzt werden.

Bevorzugt wir das Verfahren in aromatischen Kohlenwasserstoffen aus der Gruppe Benzol, Toluol, Xylole, Mesitylen, Ethylbenzol oder Amiden wie N-Methylpyrrolidon oder Ketonen wie Cyclohexanon oder Ethern aus der Gruppe, Dibutylether, Methyl-*t-*butylether, Diisopropylether, Diphenylether, Diethylenglycoldimethylether als Lösungsmittel durchgeführt.

Zur Isolierung der erfindungsgemäß hergestellten Produkte wird das Reaktionsgemisch nach Beendigung der Reaktion hydrolysiert und die Produkte durch Extraktion oder Kristallisation abgetrennt.

Zur Hydrolyse wird vorzugsweise eine wässrige Base wie z.B. wässrige Natronlauge verwendet.

### Beispiele

### Beispiele 1 - 38 zur Verfahrensvariante b):

Bei der Verfahrensvariante b) wurde der Einfluss von verschiedenen Katalysatoren sowie von Additiven bei unvollständigem Umsatz überprüft. Dafür wurde jeweils 2-Tolylsäureanhydrid (1 mmol, 128 mg) mit dem entsprechenden Katalysators (3 mol%) und der entsprechenden Menge an Additiv in NMP (1.5 mL) für 16 h bei 120 °C gerührt. Nach dem Abkühlen wurde 1-Bromopropan (0.2 mL, 271 g, 2.2 mmol, 99%), K₂CO₃ (139 mg, 1 mmol) und NMP (2 mL) zugegeben und das Reaktionsgemisch für 2-3 h auf 55 °C erhitzt. Die Ausbeuten wurden schließlich gaschromatographisch bestimmt. Es ist deutlich, dass viele Rhodiumverbindungen die Reaktion in Kombination mit verschiedenen milden Basen vermitteln und dass die besten Ergebnisse mit Rh(I)Olefin-, Carbonyl- oder Phosphinkomplexen in Kombination mit Fluorid, Carbonat und Phosphatsalzen erzielt werden.

| | | | |
|---|---|---|---|
| 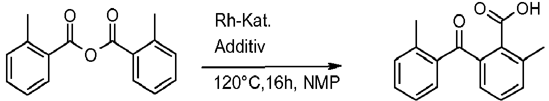 | | | |

| **Bsp.** | **Katalysator** | **Additiv (Äquiv.)** | **Ausbeute (%)** |
|---|---|---|---|
| 1 | ½ Rh₂(I)(hexadien)₂Cl₂ | KF (1) | 60 |
| 2 | ½ Rh₂(I)(cod)₂Cl₂ | " | 47 |
| 3 | Rh (I)(cod)(acac) | " | 45 |
| 4 | Rh(I)(CO)(PPh₃)₃H | " | 52 |
| 5 | Rh(I)(cod)₂BF₄ | " | 45 |
| 7 | ½ Rh₂(III)(Cp^{*})₂(Cl)₂ | " | 35 |
| 8 | ½ [Rh(II)(OAc)₂]₂ | " | 3 |
| 9 | ½ Rh₂(I)(hexdien)₂Cl₂ | NaF (1) | 1 |
| 10 | " | CsF (1) | 62 |
| 11 | " | K₂CO₃ (0.5) | 44 |
| 12 | " | Na₂CO₃ (0.5) | 50 |
| 13 | " | Cs₂CO₃ (0.5) | 47 |
| 14 | " | MnCO₃ (0.5) | 4 |
| 15 | " | ZnCO₃·Zn(OH)₂·H₂O (0.25) | 22 |
| 16 | " | K₃PO₄ (1) | 55 |
| 17 | " | K₃PO₄ (0.35) | 51 |
| 18 | " | K₂HPO₄ (0.35) | 36 |
| 19 | " | Na₃PO₄ (0.35) | 45 |
| 20 | " | LiOH H₂O (1) | 24 |
| 21 | " | KHMDS (1) | 27 |
| 22 | " | NEt₃ (1) | 5 |
| 23 | " | DBU (1) | 10 |
| 24 | " | Cy₂MeN (1) | 4 |
| 25 | " | DABCO (1) | 25 |
| 26 | " | CaH₂ (1) | 2 |
| 27 | " | NaH (1) | 38 |
| 28 | " | KF (0.5) | 51 |
| 29 | " | KF (0.2) | 43 |
| 30 | " | KF (0.1) | 41 |

### Beispiele 31 - 44 zur Verfahrensvariante a):

Gemäß der Verfahrensvariante a) wurden jeweils 2-Tolylsäure (0.5 mmol) mit Propoinsäureanhydrid (0.5 mmol), Katalysator (3 mol%) und Additiv (0.5 mmol) in Mesitylen (0.5 mL) für 16 h auf 145 °C erhitzt. Die Hydrolyse erfolgte bei 100 °C für 1 h mit 2 mL einer 6.25 M wässrigen Natriumhydroxidlösung. Die Ausbeuten wurden über HPLC bestimmt. Es ist deutlich, dass viele Rhodiumverbindungen die Reaktion in Kombination mit verschiedenen milden Basen vermitteln und dass die besten Ergebnisse mit Rh(I)Olefinkomplexen in Kombination mit Fluorid, Carbonat, Acetat und Phosphatsalzen erzielt werden.

| | | | |
|---|---|---|---|
| 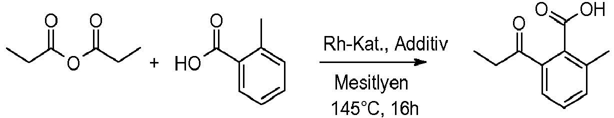 | | | |

| **Bsp.** | **Katalysator** | **Additiv** | **Ausb.** |
|---|---|---|---|
| 31 | ½ Rh₂(CP^{*})₂Cl₂ | KF (0.5) | 18 |
| 32 | RhCl₃^{*}3H₂O | " | 27 |
| 33 | ½ [Rh(II)(OAc)₂]₂ | " | 23 |
| 34 | ½ (Rh(hexadien)Cl)₂ | " | 27 |
| 35 | ½ (Rh(coe)Cl)₂ | " | 27 |
| 36 | ½ (Rh(cod)Cl)₂ | " | 29 |
| 37 | " | " | 73^{a} |
| 38 | " | NaF | 13^{a} |
| 39 | " | CsF | 82^{a} |
| 40 | " | K₃PO₄ | 72^{a} |
| 41 | " | K₂CO₃ | 71^{a} |
| 42 | " | CsOAc | 85^{a} |
| 43 | " | Cs₂CO₃ (0.25) | 93^{a} |
| 44 | " | Cs₂CO₃ (0.05) | 66^{a} |

| | | | |
|---|---|---|---|
| a) 2 mmol Propionsäureanhydrid | | | |

### Beispiel 45

**Darstellung von 2-Methyl-6-Propionylbenzoesäure:** In einem 100 mL Gefäß mit Septumkappe und Magnetrührer wurde unter Stickstoffatmosphäre, 2-Tolylsäure (1.36 g, 10.0 mmol), Cäsiumcarbonat (815 mg, 2.50 mmol) und Chloro(1,5-cyclooctadien)rhodium(I) dimer (74.0 mg, 0.15 mmol) vorgelegt. Anschließend wurden entgastes Mesitylen (10 mL) und Propionsäureanhydrid (5.13 mL, 5.21 g, 40.0 mmol) mit Hilfe von Spritzen zugesetzt und das Reaktionsgemisch für 16 Stunden auf 145 °C erhitzt. Nach dem Abkühlen wurde eine 6.25 M wässrige Natriumhydroxidlösung (40 mL) zugegeben und das Reaktionsgemisch nochmals für eine Stunde auf 100 °C erhitzt. Danach wurde die Reaktionsmischung mit konz. Salzsäure angesäuert (pH < 4) und die wässrige Phase mit Ethylacetat extrahiert (3 x 100 mL). Die organischen Phasen wurden vereinigten, mit gesättigter Kochsalzlösung gewaschen (100 mL), über Magnesiumsulfat getrocknet und das Lösungsmittel abdestilliert. Der Rückstand wurde anschließend säulenchromatographisch aufgereinigt (SiO₂, Ethylacetat / n-Hexan). Auf diese Weise wurde 2-Methyl-6- Propionylbenzoesäure (1.37 g, 7.12 mmol, 71%) als farbloser Feststoff erhalten. Schmp.: 102.6 °C..

### Beispiele 46 bis 61

Darstellung von ortho-acylierten aromatischen Carbonsäuren (3) aus aromatischen Carbonsäuren (1) und aliphatischen Carbonsäureanhydriden (2).

Analog zur Versuchsvorschrift aus Beispiel 45 wurde für die Beispiele 46-61 jeweils aromatische Carbonsäure (1) (0.5 mmol) mit Carbonsäureanhydrid (2) (2 mmol), [Rh(cod)Cl]₂ (1.5 mol%) und der entsprechenden Menge an Additiv in Mesitylen (0.5 mL) für 16 h auf 145 °C erhitzt. Die Hydrolyse erfolgte bei 100 °C für 1 h mit 2 mL einer 6.25 M wässrigen Natriumhydroxidlösung. Die Produkte wurden säulenchromatographisch aufgearbeitet (SiO₂, Ethylacetat/n-Hexan) und durch ¹H und ¹³C NMR, sowie CHNS oder HRMS charakterisiert. Die Ergebnisse sind in Tabelle 3 zusammengefasst. Die angegebenen Ausbeuten beziehen sich auf isolierte Produkte.

**Tabelle 3. Darstellung verschiedener ortho-acylierter aromatischer Carbonsäuren.**

| | | | | | | | | | |
|---|---|---|---|---|---|---|---|---|---|
| 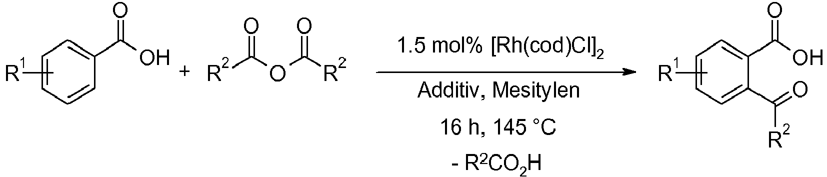 | | | | | | | | | |

| B s p | Edukte | Produkt | A dd .^{a} | Aus b. [%] | Bs p. | Edukte | Produkt | Add.^{a} | Ausb. [%] |
|---|---|---|---|---|---|---|---|---|---|
| 4 6 | R¹ = 2-Me | 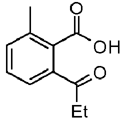 | A | 86 | 54 | R¹ = 3-Br | 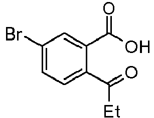 | C | 52 |
| | R² = Et | | | | | R² = Et | | | |
| 4 7 | R¹ = 3-Me | 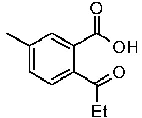 | A | 69 | 55 | R¹ = 3-NMe₂ | 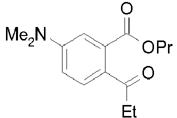 | C | 74^{b} |
| | R² = Et | | | | | R² = Et | | | |
| 4 8 | R¹ = 2,4-Me | 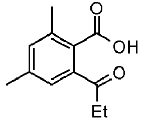 | A | 75 | 56 | R¹ = 3-C(O)Ph | 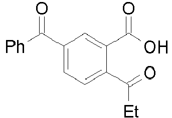 | C | 88 |
| | R² = Et | | | | | R² = Et | | | |
| 4 9 | R¹ = 2-Ph | 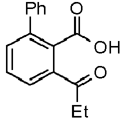 | B | 87 | 57 | 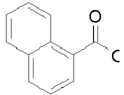 | 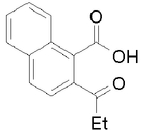 | A | 60 |
| | R² = Et | | | | | R² = Et | | | |
| 5 0 | R¹ = 2-OMe | 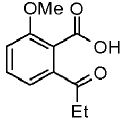 | B | 92 | 58 | 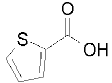 | 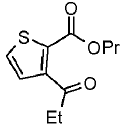 | A | 20^{b} |
| | R² = Et | | | | | R² = Et | | | |
| 5 1 | R¹ = 2,3,4-OMe | 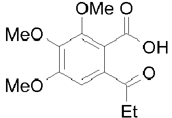 | B | 92 | 59 | R¹ = 2-Me | 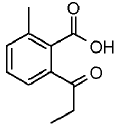 | A | 82 |
| | R² = Et | | | | | R² = n-Pentyl | | | |
| 5 2 | R¹ = 2-CF₃ | 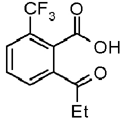 | A | 87 | 60 | R¹ = 2-Me | 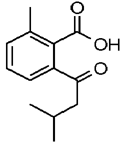 | B | 68 |
| | R² = Et | | | | | R² = Isobutyl | | | |
| 5 3 | R¹ = 3-Cl | 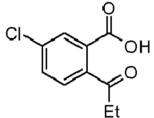 | C | 70 | 61 | R¹ = 2-Me | 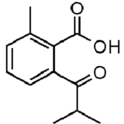 | B | 73 |
| | R² = Et | | | | | R² = Isopropyl | | | |

| | | | | | | | | | |
|---|---|---|---|---|---|---|---|---|---|
| a) A: 0.25 mmol Cs₂CO₃. B: 1 mmol KF. C: 1 mmol CsF und 155 °C. b) Aufarbeitung: 1) 10 mmol KOH, 0.4 ml H₂O, 2 mL NMP, 100 °C, 1 h; 2) 40 mmol of 1-Bromopropan, 5 mmol of K₂CO₃, 2 mL NMP, 2 h, 55 °C. | | | | | | | | | |

### Beispiele 62-65 zur Verfahrensvariante c)

Gemäß der Verfahrensvariante c) wurden jeweils aromatische Carbonsäure (1) (2 mmol) mit Pivalinsäureanhydrid (3 mmol), [Rh(cod)Cl]₂ (1.5 mol%) und KF (2 mmol) in 2 mL NMP für 16 h auf 145 °C erhitzt. Die Hydrolyse erfolgte bei 100 °C für 1 h mit 10 mmol KOH und 0.4 ml Wasser. Nach dem Abkühlen wurde zur einfachen Isolierung mit 1-Bromopropan (3.00 mL, 4.06 g, 32.7 mmol, 99%), K₂CO₃ (2.35 g, 17 mmol) und NMP (2 mL) alkyliert, wobei das Reaktionsgemisch erneut für 2-3 h auf 55 °C erhitzt wurde. Die Produkte wurden säulenchromatographisch aufgearbeitet (SiO₂, Ethylacetat/n-Hexan) und durch ¹H und ¹³C NMR, sowie CHNS oder HRMS charakterisiert. Die Ergebnisse sind in untenstehenden Graphik zusammengefasst. Die angegebenen Ausbeuten beziehen sich auf isolierte Produkte.

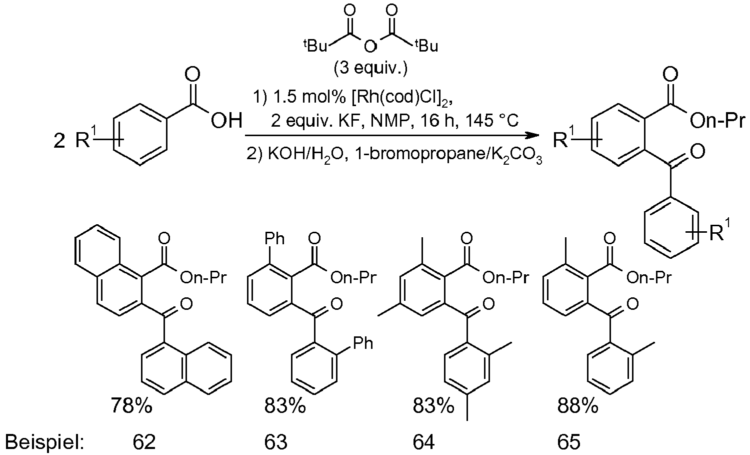

### Beispiel 66: Verwendung von Säurechlorid als Acylierungsreagenz

Unter Stickstoffatmosphäre wurde 2-Tolylsäure (68.7 mg, 0.5 mmol), Kaliumfluorid (116 mg, 2 mmol) und Chloro(1,5-cyclooctadien)rhodium(I) dimer (3.31 mg, 0.008 mmol) in einem Reaktionsgefäß vorgelegt. Anschließend wurden entgastes NMP (1.5 mL) und 2-Tolylsäurechlorid (77.3 mg, 0.5 mmol) mit Hilfe von Spritzen zugesetzt und das Reaktionsgemisch für 16 Stunden auf 160 °C erhitzt. Nach dem Abkühlen wurde zur einfachen Isolierung mit 1-Bromopropan (0.2 mL, 2 mmol), K₂CO₃ (138 mg, 1 mmol) und NMP (2 mL) alkyliert, wobei das Reaktionsgemisch erneut für 2-3 h auf 55 °C erhitzt wurde. Danach wurde die Reaktionsmischung mit Wasser verdünnt (30 mL) und mit Ethylacetat extrahiert (3 x 30 mL). Nach säulenchromatographischer Aufreinigung wurde Weise wurde 2-Toluyl-6-methylbenzoesäurepropylester (71 mg, 48%) als farbloser Öl erhalten.

## Patentansprüche

1. Verfahren zur Herstellung einer ortho-acylierten aromatischen oder heteroaromatischen Carbonsäure der Formel (3), 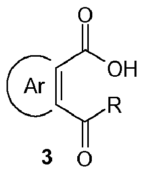 in der Ar für ein aromatisches oder heteroaromatisches Ringsystem steht,
durch Umsetzung einer Verbindung der Formel (1) 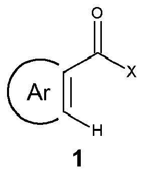 in der X für eine Hydroxylgruppe oder den Rest 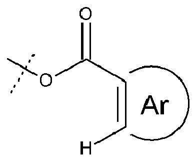 steht und Ar die für Formel (3) angegebene Bedeutung hat,
a) mit einem Acylierungsreagenz der Formel (2) 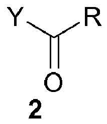 in der R für einen C₂-C₈-Alkylrest und
Y für ein Chlor-, Brom-, lod- oder Fluoratom oder einen über ein Sauerstoffatom gebundenen Carboxylrest R¹COO, bei dem R¹ unabhängig von R die gleiche Bedeutung wie R besitzt, steht
oder
b) mit einer weiteren Verbindung der Formel (1) in Form ihres Anhydrids in Gegenwart eines Rhodiumkatalysators und eines basischen Additivs.

2. Verfahren nach Anspruch 1, **dadurch gekennzeichnet , dass** das Anhydrid der Verbindung der Formel (1) in situ durch Zugabe eines Dehydratisierungsreagenz erzeugt wird.

3. Verfahren nach Anspruch 1, **dadurch gekennzeichnet, dass** das das Anhydrid der Verbindung der Formel (1) in situ durch Zugabe des Dehydratisierungsreagenz Pivalinsäureanhydrid erzeugt wird.

4. Verfahren nach einem der Ansprüche 1 bis 3 , **dadurch gekennzeichnet, dass** die Verbindung der Formel (1) eine ein oder mehrkernige C₆-C₁₄-Carbonsäure ist

5. Verfahren nach einem der Ansprüche 1 bis 3 , **dadurch gekennzeichnet, dass** die Verbindung der Formel (1) einen heteroaromatischen Kern Ar aus der Gruppe Pyridyl, Chinolinyl, Acridinyl, Pyridazinyl, Pyrimidinyl, Pyrazinyl, Triazinyl, Tetrazinyl, Pyrrolyl, Pyrazolyl, Isoxazolyl, Imidazolyl, Oxazolyl, Thiazolyl, Thiophenyl, Furanyl aufweist.

6. Verfahren nach einem der Ansprüche 1 bis 5 , **dadurch gekennzeichnet, dass** das Acylierungsreagenz der Formel (2) ein C₂-C₈-Carbonsäurechlorid oder -anhydrid ist

7. Verfahren nach einem der Ansprüche 1 bis 6 , **dadurch gekennzeichnet, dass** der Rhodiumkatalysator ein Rhodium(I) Komplex ist.

8. Verfahren nach einem der Ansprüche 1 bis 7 , **dadurch gekennzeichnet, dass** der Rhodiumkatalysator ein Katalysator aus der Gruppe 1,5-Cyclooctadien-,rhodium(I)chlorid Dimer, Di-µ-chlorobis[(1,2,5,6-η)-1,5-hexadien]dirhodium, Chloro-bis(cycloocten)rhodium(I) Dimer und Chlorobis(ethylen)rhodium(I) Dimer ist.

9. Verfahren nach einem der Ansprüche 1 bis 8 , **dadurch gekennzeichnet, dass** das basische Additiv eine anorganische Base aus der Gruppe Kaliumfluorid, Cäsiumfluorid, Lithiumcarbonat, Natriumcarbonat, Kaliumcarbonat, Cäsiumcarbonat, Kaliumhydrogencarbonat, Natriumphosphat, Kaliumphosphat, Cäsiumphosphat, Cäsiumacetat, Kaliumacetat, Kaliumhydrogenphosphat ist

10. Verfahren nach einem der Ansprüche 1 bis 9 , **dadurch gekennzeichnet, dass** der Rhodiumkatalysator in einer Menge von 0,001 bis 20 mol%, bevorzugt 0,01 bis 3 mol%, bezogen auf die Verbindung der Formel (1), eingesetzt wird.

11. Verfahren nach einem der Ansprüche 1 bis 10 , **dadurch gekennzeichnet, dass** das basische Additiv in einer Menge von 10 bis 500 mol%, bevorzugt 20 bis 200 mol%, bezogen auf die Verbindung der Formel (1), eingesetzt wird.

12. Verfahren nach einem der Ansprüche 1 bis 11 , **dadurch gekennzeichnet, dass** das Verfahren bei einer Temperatur im Bereich zwischen 20 bis 200, bevorzugt 80 bis 180, besonders bevorzugt 120 bis 155°C durchgeführt wird.
